(19) **Europäisches Patentamt / European Patent Office / Office européen des brevets**

(11) **EP 4 339 594 A1**

(12) # EUROPEAN PATENT APPLICATION
published in accordance with Art. 153(4) EPC

(43) Date of publication:
**20.03.2024 Bulletin 2024/12**

(21) Application number: **22807343.3**

(22) Date of filing: **26.04.2022**

(51) International Patent Classification (IPC):
**G01N 21/27** (2006.01)    **G01N 21/33** (2006.01)
**G01N 21/3577** (2014.01)    **G01N 21/59** (2006.01)

(52) Cooperative Patent Classification (CPC):
**G01N 21/27; G01N 21/33; G01N 21/3577;**
**G01N 21/59**

(86) International application number:
**PCT/JP2022/018880**

(87) International publication number:
**WO 2022/239641 (17.11.2022 Gazette 2022/46)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.05.2021 JP 2021081397**

(71) Applicant: **Niterra Co., Ltd.**
**Nagoya-shi, Aichi 461-0005 (JP)**

(72) Inventors:
• **TAMAI Kazusei**
  **Nagoya-shi, Aichi 461-0005 (JP)**

• **KOJIMA, Kodai**
  **Nagoya-shi, Aichi 461-0005 (JP)**
• **FUJII, Yoshiyasu**
  **Nagoya-shi, Aichi 461-0005 (JP)**
• **KOJIMA Junji**
  **Kizugawa-shi, Kyoto 619-0223 (JP)**
• **OGASAWARA Kenta**
  **Nagoya-shi, Aichi 461-0005 (JP)**
• **KUWAHARA Daiki**
  **Nagoya-shi, Aichi 461-0005 (JP)**
• **YAZAWA Katsunori**
  **Nagoya-shi, Aichi 461-0005 (JP)**

(74) Representative: **Grünecker Patent- und**
**Rechtsanwälte**
**PartG mbB**
**Leopoldstraße 4**
**80802 München (DE)**

(54) **WATER QUALITY SENSOR AND METHOD FOR MEASURING CONCENTRATION OF SUBSTANCE IN WATER**

(57)     A water quality sensor (1) includes a light source (31), a beam splitter (33), a first detector (34), a second detector (35), and a measurement space (24). The beam splitter (33) splits light emitted from the light source (31) into transmitted light and reflected light. The first detector (34) detects the transmitted light. The second detector (35) detects the reflected light. The measurement space (24) is filled with a sample of liquid. Of an optical path along which light propagates from the light source (31) to the first detector (34), a portion extending through the measurement space (24) is defined as a first optical path (L1). Of an optical path along which light propagates from the light source (31) to the second detector (35), a portion extending through the measurement space (24) is defined as a second optical path (L2). The optical path length of the second optical path (L2) differs from the optical path length of the first optical path (L1).

EP 4 339 594 A1

**(Cont. next page)**

# FIG. 6

**Description**

TECHNICAL FIELD

The present disclosure relates to a water quality sensor and to a method for measuring the concentration of a substance in water (hereinafter referred to as a "water-contained substance concentration measurement method").

BACKGROUND ART

Patent Literatures 1 and 2 disclose a technique of calculating the absorbance of a particular substance in solution on the basis of the Lambert - Beer law and determining the concentration of the above-described particular substance in the solution from the calculated absorbance.

For example, in Patent Literature 1, absorbance is obtained as follows.

In Patent Literature 1, light emitted from a light emitting diode passes through a beam splitter. The beam splitter causes a portion of the light to branch and propagate toward a reference photo detector, and the remaining light passes through a window and is introduced into a solution in a flow cell. The light having passed through the solution is detected by a photo detector.

According to the Lambert - Beer law, absorbance A is represented by the following expression (X):

$$A = -\log(I1/I0) \qquad \text{expression (X)}$$

where I0 represents the intensity of light before entering a medium, and I1 represents the intensity of light after having transmitted through the medium.

Therefore, the absorbance A is obtained by substituting the intensity of light detected by the reference photo detector and the intensity of light detected by the photo detector in expression (X) as the value of I0 and the value of I1, respectively.

CITATION LIST

PATENT LITERATURES

Patent Literature 1: JP2009-517641A
Patent Literature 2: JP2019-109054A

SUMMARY OF INVENTION

TECHNICAL PROBLEM

However, the intensity of the light detected by the reference photo detector is influenced not only by a substance which is contained in the solution and whose concentration is to be measured, but also by the turbidity of the solution. In contrast, the intensity of the light detected by the photo detector without passing through the solution is not influenced by the turbidity. Therefore, detection of absorbance on the basis of the results of detection by the reference photo detector and the photo detector raises a concern over occurrence of influence of an error stemming from thermal/electric-power-related fluctuation of a light source, lowering of light quantity, or turbidity.

The present disclosure provides a technique which can measure the concentration of a particular substance in water while suppressing the influence of an error stemming from thermal/electric-power-related fluctuation of a light source, lowering of light quantity, or turbidity.

SOLUTION TO PROBLEM

(1) A water quality sensor of the present invention comprises a light source, a beam splitter, a first detector, a second detector, and a measurement space. The above-described beam splitter splits light emitted from the above-described light source into transmitted light and reflected light. The above-described first detector detects the above-described transmitted light. The above-described second detector detects the above-described reflected light. The above-described measurement space is filled with a sample of liquid. The above-described water quality sensor determines the concentration of a particular substance in the above-described sample from a result of detection by the above-described first detector and a result of detection by the above-described second detector. Of an optical path along which light propagates from the above-described light source to the above-described first detector, a portion extending through the above-described measurement space is defined as a first optical path. Of an optical path along which light propagates from the above-described light source to the above-described second detector, a portion extending through the above-described measurement space is defined as a second optical path. An optical path length of the above-described second optical path differs from an optical path length of the above-described first optical path.

The quantity of transmitted light in a supposed state in which the emitted light enters a reference solution is assumed to be I0. Also, the quantity of light detected by the first detector in a state in which the measurement space is filled with a sample is represented by IC1, and the quantity of light detected by the second detector in a state in which the measurement space is filled with the sample is represented by IC2. Also, a splitting ratio, which is a value obtained by dividing the transmittance of the beam splitter by the reflectance thereof, is represented by D. In this case, in accordance with the Lambert - Beer law, the absorbance AC1 of light detected by the first detector is represented by the following expression (1), and the absorbance AC2 of light detected by the second detector is represented by the following expression (2).

$$AC1 = -\log(IC1/I0) \qquad \text{expression (1)}$$

$$AC2 = -\log(IC2/D{\cdot}I0) \qquad \text{expression (2)}$$

The following expression (3) is obtained from expression (1) and expression (2).

$$AC2 - AC1 = \{-\log(IC2/D{\cdot}I0)\} - \{-\log(IC1/I0)\}$$
$$= -\log(IC2/IC1) + \log D \qquad \text{expression (3)}$$

Meanwhile, in the case where the optical path length of the second optical path is N times the optical path length of the first optical path, the following expression (4) holds.

$$AC2 - AC1 = N{\cdot}AC1 - AC1$$
$$= (N - 1){\cdot}AC1 \qquad \text{expression (4)}$$

The following expression (A) is obtained from expression (3) and expression (4).

$$AC1 = [\{-\log(IC2/IC1)\} + \log D]/(N - 1) \qquad \text{expression (A)}$$

Therefore, the absorbance AC1 of light detected by the first detector can be obtained by substituting into expression (A) the splitting ratio D and the optical path length ratio N, which have been grasped beforehand, and the light quantity IC1 and the light quantity IC2 detected by the water quality sensor. Namely, this water quality sensor can obtain the absorbance AC1 without use of the light quantity I0. Therefore, it is possible to obtain the absorbance AC1 while eliminating the influence of an error which occurs due to turbidity of the sample; i.e., a difference between light quantity before entering the sample and light quantity after having passed through the sample. Thus, the concentration of the particular substance can be determined from this absorbance AC1. Accordingly, by virtue of this configuration, it is possible to measure the concentration of a particular substance in water while suppressing the influence of an error stemming from thermal/electric-power-related fluctuation of the light source, lowering of light quantity, or turbidity.

(2) The above-described water quality sensor may comprise a first transmission window, a second transmission window, and a mirror. The above-described first transmission window may separate the above-described light source and the above-described measurement space from each other. The above-described second transmission window may be disposed on a side of the above-described measurement space opposite the above-described first transmission window and separate the above-described measurement space and the above-described beam splitter from each other. The above-described mirror may reflect the above-described reflected light from the above-described beam splitter toward the above-described second detector. The above-described light source, the above-described first transmission window, and the above-described second transmission window may be disposed in a positional relationship determined such that the light emitted from light source perpendicularly impinges on surfaces of the above-described first transmission window and the above-described second transmission window. The above-described beam splitter, the above-described mirror, the above-described first transmission window, and the above-described second transmission window may be disposed in a positional relationship determined such that the above-described reflected light reflected by the above-described mirror perpendicularly impinges on surfaces of the above-described first transmission window and the above-described second transmission window.

By virtue of this configuration, it is possible to prevent a drop in transmittance at the time when the light emitted from the light source passes through the first transmission window and the second transmission window. Also, by virtue of this configuration, it is possible to prevent a drop in transmittance at the time when the light reflected by the mirror passes through the first transmission window and the second transmission window.

(3) The above-described water quality sensor may measure the concentration of nitrate ion, nitrite ion, or ammonium ion.

By virtue of this configuration, the concentration of nitrate ion, nitrite ion, or ammonium ion can be measured.

(4) The above-described water quality sensor may comprise a filter for preventing invasion of a foreign substance into the above-described measurement space.

By virtue of this configuration, it is possible to fill the measurement space with the sample while preventing invasion of a foreign substance by means of the filter.

(5) A water-contained substance concentration measurement method of the present invention comprises an installation step, an irradiation step, a splitting step, a first detection step, a second detection step, a computation step, and a determination step. In the above-described installation step, a measurement space is filled with a sample of liquid. In the above-described irradiation step, a light source is caused to emit light. In the splitting step, the light emitted from the above-described light source is split into transmitted light and reflected light by using a beam splitter. In the above-described first detection step, the above-described transmitted light is detected by using a first detector. In the above-described second detection step, the above-described reflected light is detected by using a second detector. Of an optical path along which light propagates from the above-described light source to the above-described first detector, a portion extending through the above-described measurement space is defined as a first optical path. Of an optical path along which light propagates from the above-described light source to the above-described second detector, a portion extending through the above-described measurement space is defined as a second optical path. The above-described computing step calculates an absorbance AC1 obtained from expression (A) described below.

Notably, a quantity of the above-described transmitted light detected in the above-described first detection step is represented by IC1, a quantity of the above-described reflected light detected in the above-described second detection step is represented by IC2, a splitting ratio, which is a value obtained by dividing a transmittance of the above-described beam splitter by a reflectance thereof, is represented by D, and a ratio of the optical path length of the above-described second optical path to the optical path length of the above-described first optical path is represented by N.

$$AC1 = [\{-\log(IC2/IC1)\} + \log D]/(N - 1) \qquad \text{expression (A)}$$

In the above-described determination step, the concentration of a particular substance in the above-described sample is determined from the absorbance AC1 calculated in the above-described computation step.

This water-contained substance concentration measurement method makes it possible to obtain the absorbance AC1 of light detected by the first detector, by substituting into expression (A) the splitting ratio D and the optical path length ratio N, which have been grasped beforehand, the light quantity IC1 of the transmitted light detected in the first detection step, and the light quantity IC2 of the reflected light detected in the second detection step. Namely, by virtue of this configuration, the absorbance AC1 can be obtained without use of the light quantity 10. Therefore, it is possible to obtain the absorbance AC1 while eliminating the influence of an error which occurs due to turbidity of the sample; i.e., a difference between light quantity before entering the sample and light quantity after having passed through the sample. Thus, the concentration of the particular substance can be determined from this absorbance AC 1. Accordingly, by virtue of this configuration, it is possible to measure the concentration of a particular substance in water while suppressing the influence of an error stemming from thermal/electric-power-related fluctuation of the light source, lowering of light quantity, or turbidity.

(6) The above-described irradiation step may cause the light emitted from the above-described light source to perpendicularly impinge on a surface of a first transmission window separating the above-described light source and the above-described measurement space from each other and on a surface of a second transmission window separating the above-described measurement space and the above-described beam splitter from each other. Furthermore, the above-described water-contained substance concentration measurement method may comprise a reflection step. In the above-described reflecting step, the above-described reflected light from the above-described beam splitter may be reflected toward the above-described second detector by using a mirror, and the above-described reflected light reflected by the above-described mirror may be caused to perpendicularly impinge on surfaces of the above-described first transmission window and the above-described second transmission window.

By virtue of this configuration, it is possible to prevent a drop in transmittance at the time when the light emitted from the light source passes through the first transmission window and the second transmission window. Also, by virtue of this configuration, it is possible to prevent a drop in transmittance at the time when the light reflected by the mirror passes through the first transmission window and the second transmission window.

(7) The above-described determination step may determine the concentration of nitrate ion, nitrite ion, or ammonium ion in the above-described sample by using the absorbance AC1 calculated in the above-described computation step.

By virtue of this configuration, the concentration of nitrate ion, nitrite ion, or ammonium ion can be determined.

ADVANTAGEOUS EFFECT OF INVENTION

According to the present invention, the concentration of a particular substance in water can be measured while suppressing the influence of an error stemming from thermal/electric-power-related fluctuation of a light source, lowering of light quantity, or turbidity.

BRIEF DESCRIPTION OF DRAWINGS

[FIG. 1] FIG. 1 is a configurational diagram conceptually illustrating a water quality sensor of a first embodiment.
[FIG. 2] FIG. 2 is a configurational diagram schematically illustrating a cell section of the water quality sensor of the first embodiment.
[FIG. 3] FIG. 3 is a graph of an absorption spectrum.
[FIG. 4] FIG. 4 is a partial enlarged view of FIG. 3.
[FIG. 5] FIG. 5 is a flowchart illustrating the flow of a process of determining the concentration of a particular substance.
[FIG. 6] FIG. 6 is a configurational diagram schematically illustrating a cell section of a water quality sensor of a second embodiment.

DESCRIPTION OF EMBODIMENTS

1. First embodiment

1-1. Configuration of water quality sensor 1

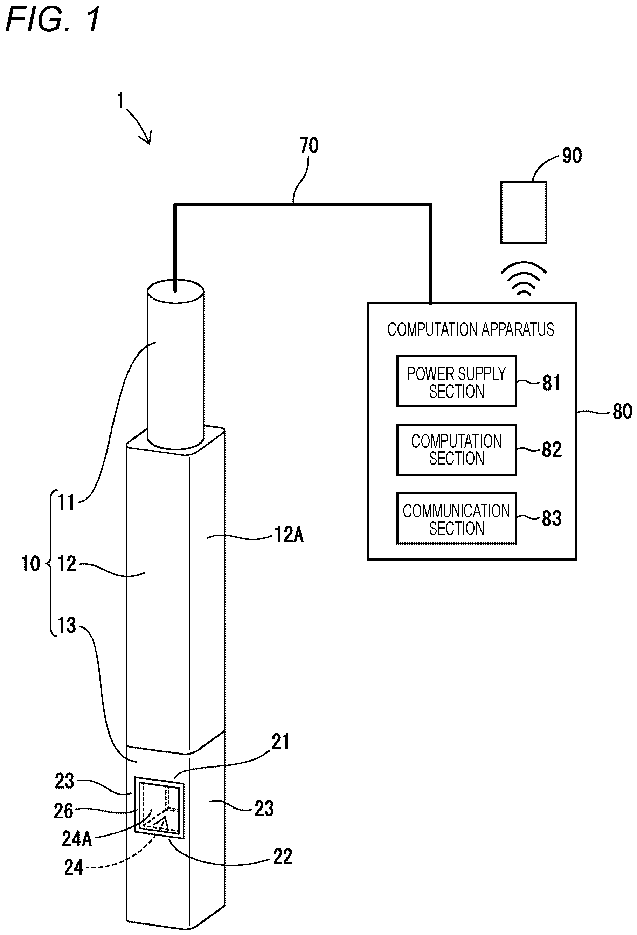

A water quality sensor 1 shown in FIG. 1 is a sensor for determining the concentration of a particular substance contained in a sample of liquid. The liquid sample is, for example, fresh water, seawater, or the like, and is a concept which encompasses liquids other than water. Preferably, the liquid sample has some degree of transparency. It is preferred that turbidity measured by, for example, a scattering-light method or a transmitted light method be 0 FTU (formazin turbidity unit) or greater and 500 FTU or less. It is sufficient that the particular substance is a light absorbing substance and is, for example, nitrate ion, nitrite ion, ammonium ion, or the like. The water quality sensor 1 determines the concentration of the particular substance by using the absorbance of the particular substance.

The water quality sensor 1 includes a sensor main body 10, a signal cable 70, and a computation apparatus 80. The sensor main body 10 has a waterproof structure. The sensor main body 10 extends in one direction. The signal cable 70 is connected to the proximal end side of the sensor main body 10. The sensor main body 10 includes a connector section 11, a body section 12, and a cell section 13, which are arranged from the proximal end side toward the distal end side of the sensor main body 10.

The signal cable 70 is connected to the proximal end side of the connector section 11, and the proximal end side of the body section 12 is connected to the distal end side of the connector section 11.

The body section 12 has a tubular body 12A. The body section 12 includes an operational amplifier, an AD convertor, an MPU, a voltage converter, etc., which are not illustrated and are disposed inside the tubular body 12A. The cell section 13 is connected to the distal end side of the tubular body 12A.

The cell section 13 is provided on the distal end side of the water quality sensor 1. The cell section 13 includes a first tube portion 21, a second tube portion 22, connecting portions 23, and a measurement space 24. The first tube portion 21 and the second tube portion 22 have a tubular shape and are coaxial with the water quality sensor 1. The first tube portion 21 and the second tube portion 22 are disposed such that they are spaced from each other in the extending direction of the water quality sensor 1. The first tube portion 21 is disposed on the proximal end side, and the second tube portion 22 is disposed on the distal end side. The first tube portion 21 and the second tube portion 22 are connected to each other by the paired connecting portions 23. The measurement space 24 is formed between the first tube portion 21 and the second tube portion 22. The measurement space 24 communicates with the outside of the water quality sensor 1. The cell section 13 has a space forming portion 24A which forms the measurement space 24. The measurement space 24 is surrounded and defined by the first tube portion 21, the second tube portion 22, and the connecting portions 23.

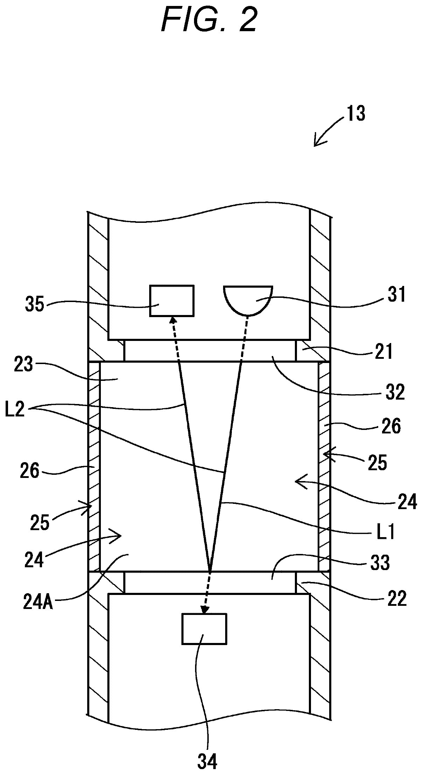

As shown in FIG. 2, the cell section 13 includes connection spaces 25 and filters 26. The connection spaces 25 are disposed on the left and right sides of the measurement space 24 in FIG. 2. The connection spaces 25 establish communication between the measurement space 24 and the outside of the water quality sensor 1. The filters 26 are provided in the connection spaces 25 and have a function of preventing invasion of foreign substances into the measurement space 24 from the outside of the water quality sensor 1. The filters 26 are disposed in pair on both sides (on the left and right sides in FIG. 2) of the measurement space 24.

The cell section 13 includes a light source 31, a transmission window 32, a beam splitter 33, a first detector 34, and a second detector 35.

The light source 31 has a function of emitting light having directivity. The light source 31 has, for example, an LED. An LED which emits light whose wavelength corresponds to a substance whose concentration is to be determined is mounted as the light source 31.

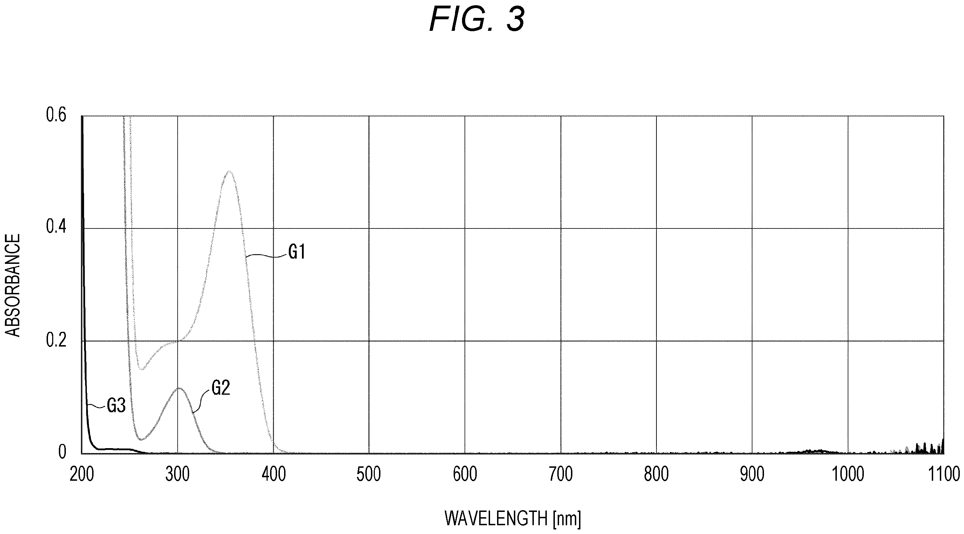

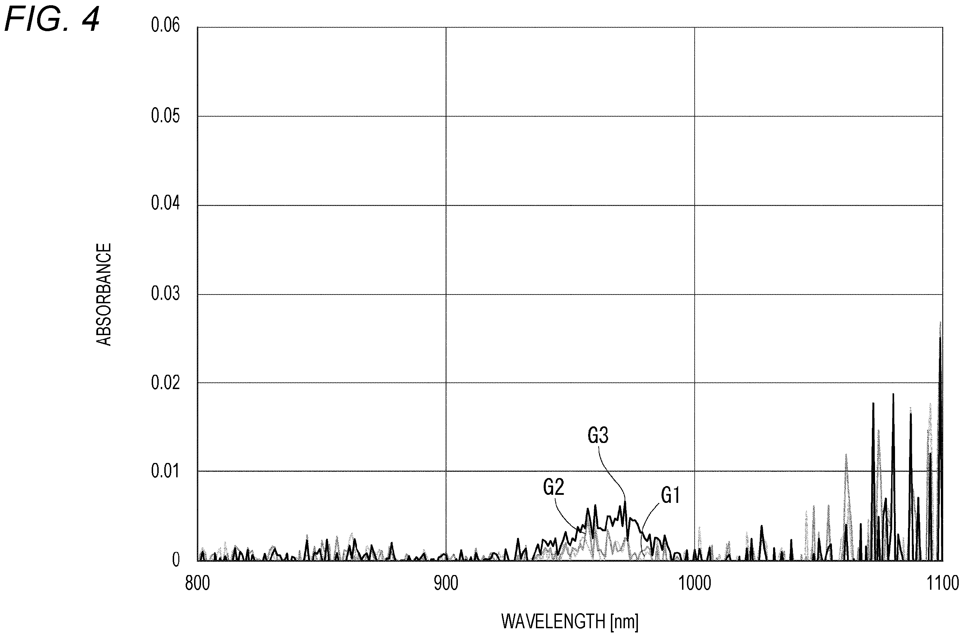

FIGS. 3 and 4 are graphs showing the relation between wavelength and absorbance. Of the curves shown in FIGS. 3 and 4, curve G1 corresponds to nitrite ion, curve G2 corresponds to nitrate ion, and curve G3 corresponds to ammonium ion.

As shown in FIG. 3, in a wavelength band from 350 nm to 400 nm (hereinafter referred to as the first wavelength band), only nitrite ion exhibits light absorption. Therefore, in the case where the concentration of nitrite ion is to be determined, an LED which emits light in the first wavelength band is mounted. Also, in a wavelength band from 260 nm to 350 nm (hereinafter referred to as the second wavelength band), nitrate ion and nitrite ion exhibit light absorption. Therefore, in the case where the concentration of nitrate ion is to be determined, an LED which emits light in the first wavelength band and an LED which emits light in the second wavelength band are mounted. Also, as shown in FIG. 4, in a wavelength band from 940 nm to 1000 nm (hereinafter referred to as the third wavelength band), nitrate ion, nitrite ion, and ammonium ion exhibit light absorption. Therefore, in the case where the concentration of ammonium ion is to be determined, an LED which emits light in the first wavelength band, an LED which emits light in the second wavelength band, and an LED which emits light in the third wavelength band are mounted. In the present embodiment, an example in which an LED which emits light in the first wavelength band, an LED which emits light in the second wavelength band, and an LED which emits light in the third wavelength band are mounted will be described. Notably, for example, in the case where the concentrations of nitrate ion and nitrite ion are known or their quantities do not produce significant differences when the concentration of ammonium ion is determined, it is sufficient that only the LED which emits light in the third wavelength band is mounted.

As shown in FIG. 2, the light source 31 is disposed on the proximal end side of the measurement space 24 and emits light toward the measurement space 24.

The transmission window 32 is a window through which the light emitted from the light source 31 passes. The transmission window 32 is, for example, a sapphire window. The transmission window 32 has a plate-like shape. The transmission window 32 is disposed in the first tube portion 21.

The beam splitter 33 has a function of splitting the light emitted from the light source 31 into transmitted light and reflected light. The transmittance TR and the reflectance RR of the beam splitter 33 may be the same or may differ from each other. Namely, a splitting ratio D, which is the value (TR/RR) obtained by dividing the transmittance TR of the beam splitter 33 by the reflectance RR thereof, may be 1 or may not be 1. The beam splitter 33 is disposed in the second tube portion 22. The beam splitter 33 is disposed on a side of the measurement space 24 opposite the transmission window 32.

Each of the first detector 34 and the second detector 35 is an element which converts light to an electrical signal, for example, a photo diode.

The light source 31, the transmission window 32, the measurement space 24, the beam splitter 33, and the first detector 34 are sequentially arranged on a straight line. Therefore, the light emitted from the light source 31 passes through the transmission window 32, the measurement space 24, and the beam splitter 33 sequentially. The transmitted light from the beam splitter 33 is detected by the first detector 34. The second detector 35 is disposed on the same side as the light source 31 (specifically, on the proximal end side) with respect to the measurement space 24. The reflected light from the beam splitter 33 passes through the transmission window 32 and is detected by the second detector 35.

Here, of an optical path along which light propagates from the light source 31 to the first detector 34, a portion which extends through the measurement space 24 is defined as a first optical path L1. Of an optical path along which light propagates from the light source 31 to the second detector 35, a portion which extends through the measurement space 24 is defined as a second optical path L2. The first detector 34 and the second detector 35 are disposed in such a manner that the optical path length of the second optical path L2 differs from the optical path length of the first optical path L1. In the present embodiment, the optical path length of the second optical path L2 is set to be two times the optical path length of the first optical path L1.

Signals representing the results of detection by the first detector 34 and the second detector 35 are amplified by the operational amplifier of the body portion 12, and the amplified signals are input to the computation apparatus 80 through the signal cable 70.

The computation apparatus 80 is connected to the sensor main body 10 via the signal cable 70, and therefore, can receive the results of detection by the first detector 34 and the second detector 35 in the sensor main body 10. The computation apparatus 80 includes a power supply section 81, a computation section 82, and a communication section 83. The power supply section 81 may be a battery or a power supply circuit for supplying to various devices electric power supplied from an external power source. The computation section 82 includes, for example, an MPU. The computation section 82 calculates the absorbance of a particular substance on the basis of the results of detection by the first detector 34 and the second detector 35, and determines the concentration of the particular substance on the basis of the calculated absorbance. The communication section 83 can communicate with an external apparatus such as terminal equipment 90. The terminal equipment 90 is a portable terminal, a personal computer, or the like.

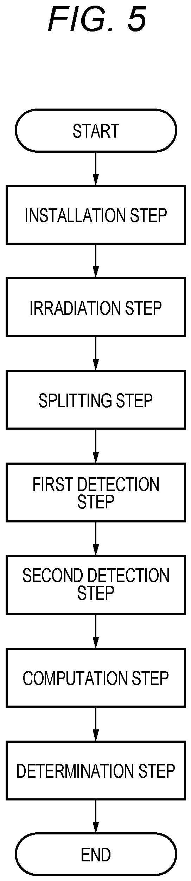

The following description relates to a method for measuring the concentration of a substance in water (hereinafter referred to as the water-contained substance concentration measurement method) which is performed by using the water quality sensor 1. As shown in FIG. 5, the water-contained substance concentration measurement method comprises an installation step, an irradiation step, a splitting step, a first detection step, a second detection step, a computation step, and a determination step.

The installation step is a step in which the measurement space 24 is filled with a liquid sample. For example, in the installation step, the sensor main body 10 of the water quality sensor 1 is thrown directly into the liquid sample. As a result, the sample flows into the measurement space 24, and the measurement space 24 is filled with the sample.

The irradiation step is a step of causing the light source 31 to emit light. When a measurement start condition is satisfied, the light source 31 emits light in response to an instruction from the computation apparatus 80. The measurement start condition is, for example, satisfaction of a time condition set beforehand or performance of a start operation by using an unillustrated operating section. The light source 31 sequentially emits light in the first wavelength band, light in the second wavelength band, and light in the third wavelength band.

The splitting step is a step of splitting the light emitted from the light source 31 into transmitted light and reflected light by using the beam splitter 33.

The first detection step is a step of detecting the transmitted light by using the first detector 34. In the first detection step, the first detector 34 detects the transmitted light and outputs a signal representing the result of the detection.

The second detection step is a step of detecting the reflected light by using the second detector 35. In the second detection step, the second detector 35 detects the reflected light and outputs a signal representing the result of the detection.

The computation step is a step which is executed by the computation apparatus 80 so as to calculate an absorbance AC1 obtained from the following expression (A).

$$AC1 = [\{-\log(IC2/IC1)\} + \log D]/(N - 1) \qquad \text{expression (A)}$$

Notably, IC 1 represents the quantity of the transmitted light detected in the first detection step. Namely, IC1 represents the quantity of light detected by the first detector 34 in a state in which the measurement space 24 is filled with the sample. IC2 represents the quantity of the reflected light detected in the second detection step. Namely, IC2 represents the quantity of light detected by the second detector 35 in a state in which the measurement space 24 is filled with the sample. D is the splitting ratio, which is a value obtained by dividing the transmittance TR of the beam splitter 33 by the reflectance RR thereof. Namely, $D = TR/RR$. N is the ratio of the optical path length of the second optical path L2 to the optical path length of the first optical path L1. Namely, $N = L2/L1$. Notably, N is a value other than 1.

Expression (A) is derived as follows.

The quantity of light before entering the sample or the quantity of light detected by the first detector 34 and the second detector 35 in a supposed state in which the measurement space 24 is filled with a reference solution is assumed to be I0. In this case, in accordance with the Lambert - Beer law, the absorbance AC1 of light detected by the first detector 34 is represented by the following expression (1), and the absorbance AC2 of light detected by the second detector 35 is represented by the following expression (2).

$$AC1 = -\log(IC1/I0) \qquad \text{expression (1)}$$

$$AC2 = -\log(IC2/D{\cdot}I0) \qquad \text{expression (2)}$$

The following expression (3) is obtained from expression (1) and expression (2).

$$AC2 - AC1 = \{-\log(IC2/D{\cdot}I0)\} - \{-\log(IC1/I0)\}$$
$$= -\log(IC2/IC1) + \log D \qquad \text{expression (3)}$$

Meanwhile, in the case where the optical path length of the second optical path L2 is N times the optical path length of the first optical path L1, the following expression (4) holds.

$$AC2 - AC1 = N{\cdot}AC1 - AC1$$
$$= (N - 1){\cdot}AC1 \qquad \text{expression (4)}$$

The following expression (A) is obtained from expression (3) and expression (4).

$$AC1 = [\{-\log(IC2/IC1)\} + \log D]/(N - 1) \qquad \text{expression (A)}$$

In the computation step, the absorbance AC1 of light detected by the first detector 34 can be obtained by substituting into expression (A) the splitting ratio D and the optical path length ratio N, which have been grasped beforehand, and the light quantity IC1 and the light quantity IC2 detected by the water quality sensor 1. Namely, this water quality sensor 1 can obtain the absorbance AC1 of light detected by the first detector 34 without use of the light quantity I0. Therefore, it is possible to obtain the absorbance AC1 while eliminating the influence of an error which occurs due to turbidity of the sample; i.e., a difference between light quantity before entering the sample and light quantity after having passed through the sample. In the computation step, the absorbance AC1 is calculated by using the above-described expression (A) for each of the first wavelength band, the second wavelength band, and the third wavelength band.

The determination step is a step which is executed by the computation apparatus 80 so as to determine the concentration of a substance in the sample by using the absorbance AC1 calculated in the computation step. Any of publicly known methods can be employed as a concrete method for determining the concentration of a substance in the sample. For example, as in the method described in Patent Literature 2, correspondence relationship data representing the relationship between absorbance and concentration are stored in advance, and the concentration of the substance is determined on the basis of the correspondence relationship data and the absorbance AC1 calculated in the computation step. The correspondence relationship data may be an arithmetic expression such as a linear function or a table.

In the determination step, the concentration of nitrite ion is determined on the basis of the absorbance AC1 corresponding to the first wavelength band. Furthermore, the overall concentration of nitrate ion and nitrite ion is determined on the basis of the absorbance AC1 corresponding to the second wavelength band, and the concentration of nitrate ion is determined by subtracting the concentration of nitrite ion from the overall concentration. Furthermore, the overall concentration of nitrate ion, nitrite ion, and ammonium ion is determined on the basis of the absorbance AC1 corresponding to the third wavelength band, and the concentration of ammonium ion is determined by subtracting the concentrations of nitrate ion and nitrite ion from the overall concentration of the three types of ions.

This method for measuring concentration in water can obtain the absorbance AC1 while eliminating the influence of an error which occurs due to turbidity of the sample; i.e., a difference between light quantity before entering the sample and light quantity after having passed through the sample. Thus, the concentration of each ion can be determined from the absorbance AC1. Accordingly, it is possible to measure the concentration of a substance in water while suppressing the influence of an error stemming from thermal/electric-power-related fluctuation of the light source, lowering of light quantity, or turbidity. In particular, by virtue of this configuration, the concentration of nitrate ion, nitrite ion, and ammonium ion can be measured.

2. Second embodiment

A water quality sensor 201 of a second embodiment differs from the water quality sensor 1 of the first embodiment in the structure of the cell section, and the structure of the remaining portion of the water quality sensor 201 is the same as that of the water quality sensor 1. In the following description, structural elements identical with those of the first embodiment are denoted by the same reference numerals, and their detailed descriptions will not be repeated.

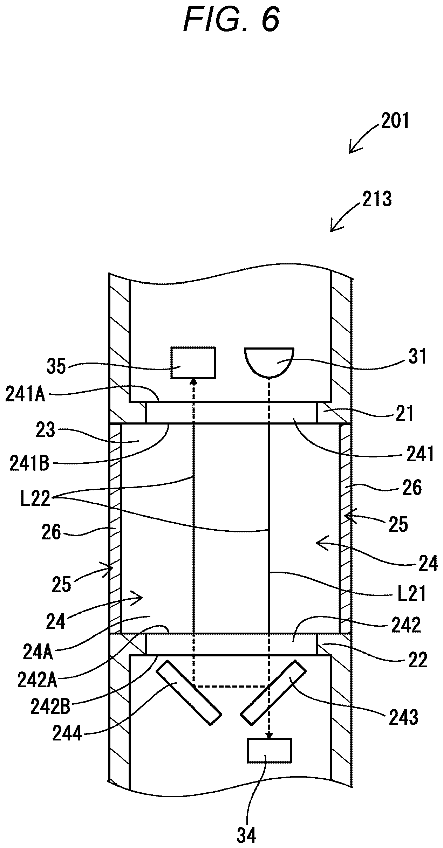

FIG. 6 shows the cell section 213 of the water quality sensor 201 of the second embodiment. The cell section 213 is provided on the distal end side of the water quality sensor 201. The cell section 213 includes a first tube portion 21, a second tube portion 22, connecting portions 23, a measurement space 24, connection spaces 25, and filters 26. The measurement space 24 communicates with the outside of the water quality sensor 201. The cell section 213 has a space forming portion 24A which forms the measurement space 24. The connection spaces 25 are disposed on the left and right sides of the measurement space 24 in FIG. 6. The connection spaces 25 establish communication between the measurement space 24 and the outside of the water quality sensor 201. The filters 26 are provided in the connection spaces 25 and have a function of preventing invasion of foreign substances into the measurement space 24 from the outside of the water quality sensor 201. The filters 26 are disposed in pair on both sides (on the left and right sides in FIG. 6) of the measurement space 24.

The cell section 213 includes a light source 31, a first transmission window 241, a second transmission window 242, a beam splitter 243, a mirror 244, a first detector 34, and a second detector 35.

The first transmission window 241 and the second transmission window 242 are windows through which the light emitted from the light source 31 passes. Each of the first transmission window 241 and the second transmission window 242 is, for example, a sapphire window. The first transmission window 241 and the second transmission window 242 have a plate-like shape.

The first transmission window 241 is disposed in the first tube portion 21. The first transmission window 241 separates the light source 31 and the measurement space 24 from each other. The second transmission window 242 is disposed in the second tube portion 22. The second transmission window 242 is disposed on a side of the measurement space 24 opposite the first transmission window 241. The second transmission window 242 separates the measurement space 24 and the beam splitter 243 from each other.

The beam splitter 243 has a function of splitting the light emitted from the light source 31 into transmitted light and reflected light. The transmittance TR and the reflectance RR of the beam splitter 243 may be the same or may differ from each other. Namely, the splitting ratio D, which is the value (TR/RR) obtained by dividing the transmittance TR of the beam splitter 243 by the reflectance RR thereof, may be 1 or may not be 1.

The mirror 244 reflects the reflected light from the beam splitter 243 toward the measurement space 24. The mirror 244 is disposed on the same side as the beam splitter 243 (specifically, on the distal end side) with respect to the measurement space 24.

The light source 31, the first transmission window 241, the measurement space 24, the second transmission window 242, the beam splitter 243, and the first detector 34 are sequentially arranged on a straight line. Therefore, the light emitted from the light source 31 passes through the first transmission window 241, the measurement space 24, the second transmission window 242, and the beam splitter 243 sequentially. The transmitted light from the beam splitter 243 is detected by the first detector 34. The second detector 35 is disposed on the same side as the light source 31 (specifically, on the proximal end side) with respect to the measurement space 24. The reflected light from the beam splitter 243 is reflected by the mirror 244. The mirror 244, the second transmission window 242, the measurement space 24, the first transmission window 241, and the second detector 35 are sequentially arranged on a straight line. The reflected light from the mirror 244 passes through the second transmission window 242, the measurement space 24, and the first transmission window 241 sequentially, and is detected by the second detector 35.

The first transmission window 241 has a first surface 241A which faces toward the light source 31 and a second surface 241B on the side opposite the first surface 241A. The second surface 241B faces toward the second transmission window 242. The second transmission window 242 has a third surface 242A which faces toward the second surface 241B of the first transmission window 241 and a fourth surface 242B on the side opposite the third surface 242A. The fourth surface 242B faces toward the beam splitter 243.

The light source 31, the first transmission window 241, and the second transmission window 242 are disposed in a positional relationship determined such that the light emitted from the light source 31 perpendicularly impinges on the first surface 241A of the first transmission window 241 and the third surface 242A of the second transmission window 242. The beam splitter 243, the mirror 244, the first transmission window 241, and the second transmission window 242 are disposed in a positional relationship determined such that the reflected light from the mirror 244 perpendicularly impinges on the second surface 241B of the first transmission window 241 and the fourth surface 242B of the second transmission window 242. Notably, the term "perpendicular" encompasses not only strictly perpendicular but also substantially perpendicular. The substantially perpendicular refers to a state in which an inclination with respect to a direction perpendicular to a surface is 3° or less.

Here, of an optical path along which light propagates from the light source 31 to the first detector 34, a portion which extends through the measurement space 24 is defined as a first optical path L21. Of an optical path along which light propagates from the light source 31 to the second detector 35, a portion which extends through the measurement space 24 is defined as a second optical path L22. The first detector 34 and the second detector 35 are disposed in such a manner that the optical path length of the second optical path L22 differs from the optical path length of the first optical path L21. In the present embodiment, the optical path length of the second optical path L22 is set to be two times the optical path length of the first optical path L21.

The following description relates to a method for measuring the concentration of a substance in water (hereinafter referred to as the water-contained substance concentration measurement method) which is performed by using the water quality sensor 201.

The water-contained substance concentration measurement method comprises a reflection step in addition to the installation step, the irradiation step, the splitting step, the first detection step, the second detection step, the computation step, and the determination step, which have been described in the first embodiment.

In the irradiation step of the second embodiment, the light emitted from the light source 31 is caused to perpendicularly impinge on the first surface 241A of the first transmission window 241 and the third surface 242A of the second transmission window 242. In the reflection step, the reflected light from the beam splitter 243 is reflected by the mirror 244, and the reflected light reflected by the mirror 244 is caused to perpendicularly impinge on the second surface 241B of the first transmission window 241 and the fourth surface 242B of the second transmission window 242.

According to the water quality sensor 201 of the second embodiment, it is possible to prevent a drop in transmittance at the time when the light emitted from the light source 31 passes through the first transmission window 241 and the second transmission window 242. Also, by virtue of this configuration, it is possible to prevent a drop in transmittance at the time when the light reflected by the mirror 244 passes through the first transmission window 241 and the second transmission window 242.

<Other embodiments>

The present invention is not limited to the embodiments described by the above description and the drawings, and, for example, the following embodiments fall within the technical scope of the present invention. Also, various features of the above-described embodiments and the following embodiments may be combined freely so long as no conflict occurs.

In the above-described embodiments, the optical path length of the second optical path is two times the optical path length of the first optical path. However, the optical path length of the second optical path is not required to be two times the optical path length of the first optical path, so long as the optical path length of the second optical path differs from the optical path length of the first optical path.

In the above-described embodiments, each of the water quality sensors has filters. However, the filters may be omitted.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

REFERENCE SIGNS LIST

    1: water quality sensor
    24: measurement space
    26: filter
    31: light source
    33: beam splitter
    34: first detector
    35: second detector
    201: water quality sensor
    241: first transmission window
    242: second transmission window
    243: beam splitter

244: mirror
AC1: absorbance of light detected by the first detector
D: splitting ratio
IC1: quantity of light detected by the first detector
IC2: quantity of light detected by the second detector
L1: first optical path
L2: second optical path
N: ratio of the optical path length of the second optical path to the optical path length of the first optical path

**Claims**

1. A water quality sensor comprising:

   a light source;
   a beam splitter for splitting light emitted from the light source into transmitted light and reflected light;
   a first detector for detecting the transmitted light;
   a second detector for detecting the reflected light; and
   a measurement space which is filled with a sample of liquid,
   the water quality sensor determining the concentration of a particular substance in the sample from a result of detection by the first detector and a result of detection by the second detector, wherein
   of an optical path along which light propagates from the light source to the first detector, a portion extending through the measurement space is defined as a first optical path;
   of an optical path along which light propagates from the light source to the second detector, a portion extending through the measurement space is defined as a second optical path; and
   an optical path length of the second optical path differs from an optical path length of the first optical path.

2. A water quality sensor according to claim 1, further comprising:

   a first transmission window which separates the light source and the measurement space from each other;
   a second transmission window which is disposed on a side of the measurement space opposite the first transmission window and separates the measurement space and the beam splitter from each other; and
   a mirror for reflecting the reflected light from the beam splitter toward the second detector, wherein
   the light source, the first transmission window, and the second transmission window are disposed in a positional relationship determined such that the light emitted from light source perpendicularly impinges on surfaces of the first transmission window and the second transmission window; and
   the beam splitter, the mirror, the first transmission window, and the second transmission window are disposed in a positional relationship determined such that the reflected light reflected by the mirror perpendicularly impinges on surfaces of the first transmission window and the second transmission window.

3. A water quality sensor according to claim 1 or 2, wherein the concentration of nitrate ion, nitrite ion, or ammonium ion is measured.

4. A water quality sensor according to claim 3, further comprising a filter for preventing invasion of a foreign substance into the measurement space.

5. A water quality sensor according to claim 1 or 2, further comprising a filter for preventing invasion of a foreign substance into the measurement space.

6. A method for measuring the concentration of a substance in water, comprising:

   an installation step of filling a measurement space with a sample of liquid;
   an irradiation step of causing a light source to emit light;
   a splitting step of splitting the light emitted from the light source into transmitted light and reflected light by using a beam splitter;
   a first detection step of detecting the transmitted light by using a first detector;
   a second detection step of detecting the reflected light by using a second detector;
   a computing step of calculating an absorbance AC1 obtained from the following expression (A):

$$AC1 = [\{-\log(IC2/IC1)\} + \log D]/(N - 1) \qquad \text{expression (A)}$$

where IC1 represents a quantity of the transmitted light detected in the first detection step, IC2 represents a quantity of the reflected light detected in the second detection step, D is a splitting ratio, which is a value obtained by dividing a transmittance of the beam splitter by a reflectance thereof, and N is a ratio of an optical path length of a second optical path to an optical path length of a first optical path, the first optical path being a portion of an optical path along which light propagates from the light source to the first detector, the portion extending through the measurement space, the second optical path being a portion of an optical path along which light propagates from the light source to the second detector, the portion extending through the measurement space; and

a determination step of determining the concentration of a particular substance in the sample by using the absorbance AC1 calculated in the computation step.

7. A method for measuring the concentration of a substance in water according to claim 6, wherein the irradiation step causes the light emitted from the light source to perpendicularly impinge on a surface of a first transmission window separating the light source and the measurement space from each other and on a surface of a second transmission window separating the measurement space and the beam splitter from each other, and

wherein the method further comprises a reflection step of reflecting the reflected light from the beam splitter toward the second detector by using a mirror, and causing the reflected light reflected by the mirror to perpendicularly impinge on surfaces of the first transmission window and the second transmission window.

8. A method for measuring the concentration of a substance in water according to claim 6 or 7, wherein the determination step determines the concentration of nitrate ion, nitrite ion, or ammonium ion in the sample by using the absorbance AC1 calculated in the computation step.

# FIG. 1

# FIG. 2

EP 4 339 594 A1

# FIG. 3

FIG. 4

# FIG. 5

```
        ┌─────────────┐
        │    START    │
        └──────┬──────┘
               ↓
    ┌──────────────────────┐
    │  INSTALLATION STEP    │
    └──────────┬───────────┘
               ↓
    ┌──────────────────────┐
    │   IRRADIATION STEP    │
    └──────────┬───────────┘
               ↓
    ┌──────────────────────┐
    │    SPLITTING STEP     │
    └──────────┬───────────┘
               ↓
    ┌──────────────────────┐
    │    FIRST DETECTION    │
    │         STEP          │
    └──────────┬───────────┘
               ↓
    ┌──────────────────────┐
    │   SECOND DETECTION    │
    │         STEP          │
    └──────────┬───────────┘
               ↓
    ┌──────────────────────┐
    │   COMPUTATION STEP    │
    └──────────┬───────────┘
               ↓
    ┌──────────────────────┐
    │  DETERMINATION STEP   │
    └──────────┬───────────┘
               ↓
        ┌─────────────┐
        │     END     │
        └─────────────┘
```

# FIG. 6

**EP 4 339 594 A1**

# INTERNATIONAL SEARCH REPORT

International application No.

**PCT/JP2022/018880**

### A. CLASSIFICATION OF SUBJECT MATTER

*G01N 21/27*(2006.01)i; *G01N 21/33*(2006.01)i; *G01N 21/3577*(2014.01)i; *G01N 21/59*(2006.01)i
FI: G01N21/27 D; G01N21/33; G01N21/59 C; G01N21/3577

According to International Patent Classification (IPC) or to both national classification and IPC

### B. FIELDS SEARCHED

Minimum documentation searched (classification system followed by classification symbols)

G01N21/00-21/01; G01N21/17-21/61

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | US 2010/0128256 A1 (THOMSON, A. I.) 27 May 2010 (2010-05-27) | 1, 3-5 |
| | paragraphs [0001]-[0033], fig. 1 | |
| Y | paragraphs [0001]-[0033], fig. 1 | 1, 3-6, 8 |
| X | JP 1-313737 A (CANON INC) 19 December 1989 (1989-12-19) | 1, 3-5 |
| | p. 1, right column, line 18 to p. 3, left column, line 6, fig. 4 | |
| Y | p. 1, right column, line 18 to p. 3, left column, line 6, fig. 4 | 2, 6-8 |
| Y | JP 2008-70293 A (YOKOGAWA ELECTRIC CORP) 27 March 2008 (2008-03-27) | 1-8 |
| | paragraphs [0001], [0016]-[0028], fig. 1 | |
| Y | JP 11-503236 A (ALFA LAVAL AB) 23 March 1999 (1999-03-23) | 1-8 |
| | p. 8, the last line to p. 27, line 17, fig. 1b | |
| Y | JP 2004-294355 A (CITIZEN WATCH CO LTD) 21 October 2004 (2004-10-21) | 2-5, 7-8 |
| | paragraphs [0013]-[0015], fig. 1 | |
| Y | JP 10-108857 A (HITACHI LTD) 28 April 1998 (1998-04-28) | 6-8 |
| | paragraphs [0003]-[0007] | |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 June 2022** | **21 June 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**
Information on patent family members

International application No.

**PCT/JP2022/018880**

| Patent document cited in search report | | | Publication date (day/month/year) | Patent family member(s) | | | Publication date (day/month/year) |
|---|---|---|---|---|---|---|---|
| US | 2010/0128256 | A1 | 27 May 2010 | WO 2009/007699 A1 p. 1, line 2 to p. 7, line 30, fig. 1 EP 2012110 A1 CA 2688625 A1 CN 101688828 A | | | |
| JP | 1-313737 | A | 19 December 1989 | (Family: none) | | | |
| JP | 2008-70293 | A | 27 March 2008 | (Family: none) | | | |
| JP | 11-503236 | A | 23 March 1999 | US 6315955 B1 column 2, line 25 to p. 14, line 57, fig. 1b WO 1996/031764 A1 EP 1710556 A2 | | | |
| JP | 2004-294355 | A | 21 October 2004 | (Family: none) | | | |
| JP | 10-108857 | A | 28 April 1998 | (Family: none) | | | |

Form PCT/ISA/210 (patent family annex) (January 2015)

**REFERENCES CITED IN THE DESCRIPTION**

**Patent documents cited in the description**

- JP 2009517641 A

- JP 2019109054 A